(19) Europäisches Patentamt
European Patent Office
Office européen des brevets

(11) Veröffentlichungsnummer: **0 078 532**
**B1**

(12) # EUROPÄISCHE PATENTSCHRIFT

(45) Veröffentlichungstag der Patentschrift: **07.01.87**

(51) Int. Cl.⁴: **C 07 D 501/36**, A 61 K 31/545

(21) Anmeldenummer: **82110072.4**

(22) Anmeldetag: **02.11.82**

(54) **Kristalline Salze von Cefodizim und Verfahren zu ihrer Herstellung.**

(30) Priorität: **03.11.81 DE 3143537**

(43) Veröffentlichungstag der Anmeldung: **11.05.83 Patentblatt 83/19**

(45) Bekanntmachung des Hinweises auf die Patenterteilung: **07.01.87 Patentblatt 87/2**

(84) Benannte Vertragsstaaten: **AT BE CH DE FR GB IT LI LU NL SE**

(56) *Entgegenhaltungen:*
**FR - A - 2 385 722**

**UNLISTED DRUGS, Band 33, Nr. 11, November 1981, Seite 174, sub e; UNLISTED DRUGS, Band 33, Nr. 9, September 1981, Seite 138, sub d Encyclopedia of Chemical Technology, Kirk-Othmer, 3rd Edition, Vol. 7, pp. 261-262, John Wiley&Sons**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Scheunemann, Karl-Heinz, Dr., Geisenheimer Strasse 88, D-6000 Frankfurt am Main 71 (DE)**
Erfinder: **Mencke, Burkhard, Dr., Im Güldenstück 13, D-6270 Idstein/Taunus (DE)**
Erfinder: **Blumbach, Jürgen, Dr., Merziger Weg 1a, D-6000 Frankfurt am Main 71 (DE)**
Erfinder: **Dürckheimer, Walter, Dr., Im Lerchenfeld 45, D-6234 Hattersheim am Main (DE)**
Erfinder: **Fleischmann, Klaus, Dr., Winkelgasse 8, D-6500 Mainz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gegenstand der Erfindung sind kristalline, wasserlösliche Salze der 7-β-[2-(2-Aminothiazol-4-yl)-2-syn-methoximinoacetamido]-3-(5-carboxymethyl-4-methyl-1,3-thiazol-2-yl-thiomethyl)-ceph-2-em-4-carbonsäure der Formel I,

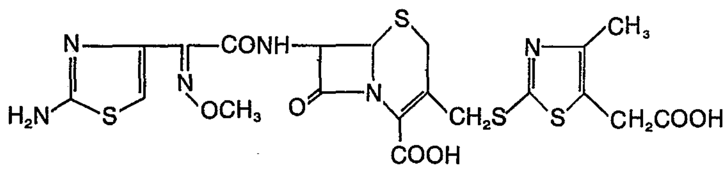

(I)

die auch die generische Bezeichnung Cefodizim trägt, sowie ein Verfahren zu ihrer Herstellung.

Cefodizim (vgl. BE 865 632) besitzt neben einer hohen antibakteriellen Aktivität und ausgeprägter β-Laktamasestabilität eine deutlich verlängerte Eliminationshalbwertszeit. Diese Eigenschaften machen es zu einem wertvollen Antibiotikum. Parenteral ist Cefodizim wegen seiner ausserordentlich geringen Wasserlöslichkeit nicht anwendbar.

Demgegenüber sind seine amorphen und kristallinen, wasserlöslichen Salze für die parenterale Anwendung besonders gut geeignet. Die kristallinen, wasserlöslichen Salze des Cefodizims sind in der belgischen Patentschrift 865 632 nicht beschrieben.

Aus einer Reihe von Gründen ist es erstrebenswert, die Salze des Cefodizims in einer kristallinen Form zu erhalten. So wird im allgemeinen mit der Kristallisation eine hohe Reinheit des Produktes erreicht. Auch ist es auf diesem Weg möglich, in den Fällen, in denen Lösungsmittel oder andere bei der Salzherstellung verwendete Stoffe in das Kristallgitter eingelagert oder absorbiert werden, Produkte definierter oder reproduzierbarer Zusammensetzung zu erhalten. Das Produkt kann so leicht standardisiert werden. Kristalline Stoffe sind weiterhin besser handzuhaben als amorphe. Dieses spielt besonders bei der Isolierung des Produktes, z.B. durch Filtration oder Schleudern eine grosse Rolle. Aber auch die Abfüllbarkeit wird beispielsweise durch die erhöhte Rieselfähigkeit erleichtert.

Die Aufgabe der Erfindung war daher die Herstellung von kristallinen, wasserlöslichen Salzen des Cefodizims der Formel I, sowie von deren Addukten bzw. Solvaten mit Wasser oder organischen Lösungsmitteln.

Die in der Patent- und sonstiger Literatur beschriebenen Verfahren zur Herstellung kristalliner Salze der verschiedensten Cephalosporine führten bei ihrer Anwendung auf Cefodizim nicht zum gewünschten Ergebnis. So konnte das in der DE-OS 2 708 439 beschriebene Verfahren zur Herstellung eines kristallinen Natriumsalzes von Cefotaxim nicht mit Erfolg angewandt werden. Bei diesem Verfahren tritt Kristallisation des Salzes aus methanolischer Lösung bei Raumtemperatur ein. Die Löslichkeit des Dinatriumsalzes von Cefodizim ist jedoch im Vergleich zu Natriumsalzen anderer Cephalosporine ausserordentlich hoch.

Auch das in der DE-OS 2 614 668 beschriebene spezielle Gefriertrocknungsverfahren zur Darstellung kristalliner Natriumsalze von z.B. Cephalordin, Cephalothin oder Cefazolin führte nur zu einem amorphen Dinatriumsalz des Cefodizims.

Bei diesem Stand der Technik war es nicht zu erwarten, dass Versuche, Cefodizim in hervorragend kristallisierende Salze zu überführen, Erfolg haben würden.

Es wurde nun gefunden, dass man kristalline, wasserlösliche Salze des Cefodizims dann erhält, wenn man die Säure der Formel I mit mindestens dem zweifachen Äquivalent einer basischen Verbindung in Wasser in Lösung bringt, diese Lösung mit einem mit Wasser mischbaren, organischen Lösungsmittel, wobei Methanol ausgenommen ist, versetzt, bis Kristallisation eintritt und – falls erwünscht – aus den so erhaltenen Addukten und Solvaten das organische Lösungsmittel und gegebenenfalls auch das Wasser entfernt.

Die basische Verbindung kann in geringem Überschuss, vorzugsweise jedoch in einer Menge von 2 Mol, bezogen auf ein Mol Cefodizim zur Anwendung kommen.

Geeignet zur Darstellung der erfindungsgemässen Salze sind anorganische und organische Basen, die als Kationen Alkalikationen, wie beispielsweise Lithium, Natrium oder Kalium, bevorzugt Natrium und Kalium, besonders bevorzugt jedoch Natrium, Erdalkalikationen, wie beispielsweise Magnesium und Calcium, das Ammonium sowie substituierte Ammoniumionen, wie beispielsweise Di- oder Tri-ethylammonium enthalten. Anionen dieser Basen können das Hydroxylion, das Hydrogencarbonation, das Carbonation oder das Anion einer organischen Säure mit 1 bis 8, vorzugsweise 1–4 C-Atomen sein, wie beispielsweise Formiat, Acetat, Propionat, α-Methylpropionat, 2-Ethylhexanoat, aber auch Anionen der allgemeinen Formel $RO^{\ominus}$, in der R für Alkyl mit 1–4 C-Atomen steht, wie beispielsweise Methyl, Ethyl, n-Propyl, Isopropyl, n-Butyl, Isobutyl, 2-Butyl oder tert.-Butyl, vorzugsweise Methyl und Ethyl. Gut geeignete Kationen können aber auch basische Aminosäuren sein, wie beispielsweise Lysin oder Arginin in ihrer protonierten Form.

Als organische, mit Wasser mischbare Lösungsmittel kommen z.B. in Betracht Ethanol, Isopropanol, Propanol, Butanol, 2-Butanol, Isobutanol, tert.-Butanol, Aceton, Tetrahydrofuran, 1,4-Dioxan oder Gemische derselben. Methanol kommt wegen der vorstehend erwähnten, ungünstigen Lösungsverhältnisse nicht in Betracht. Besonders bevorzugt sind Ethanol, Propanol, Isopropanol und Aceton. Zur Vervollständigung der Kristallisation kann auch ein mit Wasser nicht mischbares Lösungsmittel, wie beispielsweise Diethylether, Diisopropylether und Toluol zu der Suspension der Kristalle in dem Gemisch aus Wasser und dem mit Wasser mischbaren Lösungsmittel gegeben werden.

Die Konzentration der wässrigen Lösung an dem Salz des Cefodizims, das erfindungsgemäss durch Zugabe eines organischen Lösungsmittels zur Kristallisation gebracht werden soll, liegt zweckmässig zwischen etwa 5 und 50%, vorzugsweise zwischen etwa 10 und 30%.

Die Menge des insgesamt einzusetzenden organischen Lösungsmittels bzw. Lösungsmittelgemisches beträgt bis zum etwa zwanzigfachen des Volumens der wässrigen Lösung. Auch die Anwendung einer noch grösseren Menge ist möglich, bringt in der Regel jedoch keine besonderen Vorteile. Die Vereinigung der wässrigen Lösung mit dem organischen Lösungsmittel bzw. Lösungsmittelgemisch sollte langsam, z.B. tropfenweise, erfolgen, um eine gute Kristallinität und damit auch eine hohe Reinheit des Produktes zu erreichen.

Zweckmässigerweise verläuft die Kristallisation bei Raumtemperatur. Aber auch bei Temperaturen von beispielsweise 0 bis 60 °C erhält man gute Ergebnisse. Eine Nachrührzeit von bis zu etwa 3 Stunden oder mehr vervollständigt die Kristallisation.

Die so erhaltenen Kristalle des Cefodizimsalzes werden nach üblichen Laborverfahren, wie z.B. Filtration, wenn gewünscht unter sterilen Bedingungen, abgetrennt und unter schwachem Vakuum von anhaftendem Lösungsmittel befreit. Sie enthalten als Addukte bzw. Solvate pro Mol Salz noch bis zu 2 Mol Wasser und bis zu 2 Mol organisches Lösungsmittel.

Die Entfernung des organischen Lösungsmittel aus dem kristallinen Produkt kann auf verschiedenen Wegen erfolgen. So ist es möglich, das organische Solvens entweder an der Luft (mit ausreichendem Feuchtigkeitsgehalt, d.h. > 50%) oder in einem geschlossenen Gefäss unter feuchter Atmosphäre gegen Wasser auszutauschen. Die Wasseraufnahme ist in der Regel nach etwa 12 bis 72 Stunden vollständig. Die so behandelten Kristalle enthalten kein organisches Solvens mehr und weisen einen Wassergehalt von 3 bis 3.5 Mol Wasser pro Mol Salz auf.

Setzt man die durch Filtration gewonnenen Kristalle einem Hochvakuum (< 1 Torr) aus, wird sowohl organisches Lösungsmittel als auch Wasser, besonders in Gegenwart eines Trocknungsmittels, wie z.B. konzentrierter Schwefelsäure, Phosphorsäureanhydrid, aber auch Ätzkali oder Ätznatron, sowie Silicagel (Blaugel) entfernt.

Eine solche Probe nimmt wieder bis zu 3 bis 3.5 Mol Wasser pro Mol Salz auf, wenn man sie Bedingungen unterwirft, wie sie vorstehend zur Entfernung organischer Lösungsmittel aus dem kristallinen Produkt beschrieben wurden, ohne jedoch ihre weiter unten definierten kristallinen Eigenschaften zu verlieren. Durch Einwirkung eines organischen Lösungsmittels kann ein kristallines, wasser- und lösungsmittelfreies Salz auch in ein Solvat überführt werden.

Mit dem kristallinen Natriumsalz des Cefodizims unterschiedlichen Wasser- und Solvensgehalts durchgeführte Stabilitätsuntersuchungen zeigen, dass besonders nahezu wasserfreie Chargen bei z.B. einmonatiger Lagerung bei 60 °C praktisch keine Abnahme der antibiotischen Wirksamkeit aufweisen, womit der positive Einfluss eines definierten Wasser- und Solvensgehalts und einer definierten Kristallinität auf die Stabilität des Salzes erwiesen wurde.

Die folgenden Beispiele sollen die Erfindung näher erläutern, ohne sie jedoch auf die angeführten Beispiele zu beschränken.

Beispiel 1

Di-natriumsalz der 7-β-[2-(2-Aminothiazol-4-yl)-2-syn-methoximino-acetamido]-3-(5-carboxymethyl-4-methyl-1,3-thiazol-2-yl-thiomethyl)-ceph-3-em-4-carbonsäure (Di-natriumsalz von Cefodizim)

46,0 g Cefodizim wurden in 100 ml Wasser suspendiert, mit 13,2 g wasserfreiem Natriumbicarbonat versetzt und bis zur vollständigen Auflösung bei Raumtemperatur gerührt. Die schwach gelbgefärbte Lösung wird mit 2 g Filterkohle aufgerührt, filtriert, das Filtrat mit Wasser auf 150 ml verdünnt. Zu dieser Lösung tropft man bei 20–25 °C unter ständigem Rühren 1000 ml Ethanol. Nach vollständiger Zugabe des Ethanols wird 1 Stunde unter Eiskühlung nachgerührt und dann abfiltriert. Man erhält 34,1 g Di-natriumsalz des Cefodizims in Form farbloser Kristalle.

Analyse:

| | |
|---|---|
| Na<br>(aus der Sulfatasche) | ber. 6,4%<br>gef. 6,4% |
| $H_2O$<br>(Karl Fischer Methode) | ber. 3.7 (1.5 Mol)<br>gef. 3.8 |
| Ethanol<br>(Gaschromat.) | ber. 9,5 (1,5 Mol)<br>gef. 8,5 |

IR: $\nu_{\beta\text{-Laktam}}$: 1775 $cm^{-1}$

$^1H$-NMR ($d_6$-DMSO): $\delta$ = 2,14 (s; $CH_2$-Thiazol)
3.23 (s; $CH_2$-COOH und Ceph-(-2-$CH_2$)
3.26 (" " " " )
3.83 (s; N-O-$CH_3$)
4.03, 4.51 (AB-System; -$CH_2$-S; J = 13 Hz)
4.92 (d; C-6-H;) J = 5 Hz
5.03 (q; C-7-H; J = 5Hz J = 7 Hz
Resonanzsign. von
Ethanol: 1.04; t; $CH_3$
3.41; q; $CH_2$
6.66 (s; C-5-Thiazol-H)
7.02 (breites s; $NH_2$)
9.42 (d; NHCO; J = 7 Hz)

Röntgenbeugungsdiagramm (Ni-gefilterte Kupferstrahlung; λ = 1,5418 Å)

| Netzebenenabstand d | rel. Intensität ($J/J_{max}$) |
|---|---|
| 8,66 | 1,00 |
| 7,63 | 0,43 |
| 7,25 | 0,36 |
| 6,57 | 0,16 |
| 6,32 | 0,49 |
| 5,37 | 0,17 |
| 5,22 | 0,45 |
| 4,96 | 0,35 |
| 4,67 | 0,09 |
| 4,55 | 0,33 |
| 4,33 | 0,46 |
| 4,10 | 0,98 |
| 3,98 | 0,45 |
| 9,90 | 0,40 |
| 3,81 | 0,42 |
| 3,79 | 0,43 |
| 3,73 | 0,40 |
| 3,62 | 0,10 |
| 3,47 | 0,26 |
| 3,39 | 0,43 |
| 3,24 | 0,17 |
| 3,15 | 0,06 |
| 3,08 | 0,18 |
| 2,86 | 0,09 |

Aus einer solchen Probe wird Ethanol durch Stehenlassen an Luft ausreichender Feuchtigkeit für 12–16 h entfernt. NMR-spektroskopisch ist dann kein Alkohol nachzuweisen, die GC-Analyse zeigt maximal 1%, die durch Verlängern der Prozedur ebenfalls entfernt werden können.

Beispiel 2
Di-natriumsalz von Cefodizim

51,0 g Cefodizim werden mit 14,8 g Natriumhydrogenkarbonat in 220 ml Wasser gelöst. Diese Lösung tropft man bei Raumtemperatur langsam in 4350 ml Isopropanol. Aus der zunächst milchig trüben Suspension fallen nach beendeter Zugabe farblose Kristalle aus. Man lässt noch 4 Stunden nachrühren, saugt ab und trocknet über Nacht. Nach weiteren 8 Stunden bei 40 °C/150 Torr über Silicagel (Blaugel) erhält man 54 g des Di-natriumsalzes des Cefodizims in Form farbloser Kristalle.

IR: $\nu_{\beta\text{-Laktam}}$ : 1775 $cm^{-1}$

Beispiel 3
Di-kaliumsalz von Cefodizim

5,84 g Cefodizim werden mit 2,0 g Kaliumhydrogencarbonat in 30 ml Wasser gelöst und die Lösung filtriert. Man fügt bei 20–25 °C 300 ml Isopropanol zur Lösung und rührt 1 Stunde unter Eiskühlung nach. Filtration und Trocknen über KOH ergibt 3,4 g des Di-kaliumsalzes des Cefodizims in Form farbloser Kristalle.

Analyse:

| | | |
|---|---|---|
| $H_2O$ (Karl-Fischer Methode) | ber. 1,1% (für o,5 Mol) | gef. 1,1% |
| Isopropanol: (Gaschromatograph) | ber. 4,3% (für 0,5 Mol) | gef. 5,4% |
| Kalium (aus der Sulfatasche) | ber. 11,1% | gef. 10,7% |

IR: $\nu_{\beta\text{-Laktam}}$ : 1772

Rötgenbeugungsdiagramm (Ni-gefilterte Kupferstrahlung $\lambda$ = 1,5418 Å)

| Netzebenenabstand d | rel. Intensität ($J/J_{max}$) |
|---|---|
| 18,01 | 0,98 |
| 12,96 | 0,81 |
| 9,98 | 0,29 |
| 8,51 | 0,30 |
| 8,25 | 0,53 |
| 7,15 | 0,27 |
| 6,63 | 0,19 |
| 6,04 | 0,15 |
| 5,66 | 0,27 |
| 5,41 | 0,52 |
| 5,21 | 0,28 |
| 5,01 | 0,16 |
| 4,80 | 0,18 |
| 4,60 | 0,31 |
| 4,59 | 0,39 |
| 4,52 | 0,43 |
| 4,36 | 0,45 |
| 4,27 | 0,69 |
| 4,11 | 0,86 |
| 4,04 | 1,00 |
| 3,92 | 0,80 |
| 3,72 | 0,61 |
| 3,64 | 0,64 |
| 3,43 | 0,54 |
| 3,13 | 0,44 |
| 2,97 | 0,43 |

Das als Ausgangsmaterial eingesetzte Cefodizim kann auf folgende Weise erhalten werden.

6,1 g 2-(2-Mercapto-4-methyl-1,3-thiazol-5-yl)-essigsäure werden in 75 ml Wasser suspendiert und mit 22 ml 2n Natronlauge auf pH 6,5 eingestellt. Man erwärmt auf 70 °C und lässt innerhalb von 2 h bei dieser Temperatur eine Lösung aus 11,9 g Cefotaxim in 75 ml Wasser zutropfen. Es wird 2 Stunden nachgerührt (bei 70 °C!) und durch Zugabe von 2n NaOH Lösung konstant auf pH 6,5 gehalten (Verbrauch: ca. 9 ml 2n NaOH-Lösung).

Schliesslich kühlt man auf 25 °C, fügt ca. 38 ml 2n HCl Lösung (pH sinkt auf 2,8) und kühlt bis 0 °C. Der Niederschlag wird abgesaugt, mit 200 ml Wasser ausgerührt, erneut abgesaugt und mit weiteren 100 ml Wasser gewaschen. Nach Trocknen im Hochvakuum über $P_4O_{10}$ bei ca. 20 °C erhält man 10,5 g Cefodizim als hellbraun gefärbtes Produkt.

100 g des so erhaltenen Cefodizims wurden fein gepulvert, in 300 ml Wasser suspendiert und mit Natriumhydrogencarbonat in Lösung gebracht. Mit verdünnter Salzsäure wurde der pH-Wert der Lösung auf etwa 6,5 eingestellt, die durch Schwebstoffe schwach getrübte Lösung filtriert und das klare, braun gefärbte Filtrat auf eine Säule gegeben, die wie folgend beschrieben, vorbereitet worden war:

1 kg Polystyroladsorptionsharz HP 20 (auch XAD2 u.ä. sind geeignet) liess man mit Methanol quellen, füllte diese Menge in eine Chromatographiersäule 45 × 10 cm und wusch mit Wasser methanolfrei.

Die aufgetragene Lösung des rohen Natriumsalzes des Cefodizims eluierte man mit 6 l Wasser. Die ersten 1,6 l umfassenden Fraktionen enthielten kein Produkt und wurden verworfen. Das übrige Eluat wurde unter Rühren auf pH 5 angesäuert, mit einigen Impfkristallen versetzt und langsam, unter Beibehaltung der Umgebungstemperatur, bis auf pH 2,8 angesäuert. Man liess noch weitere 30 Minuten nachrühren und filtrierte ab. Ausbeute nach erschöpfendem Trocknen über Phosphorpentoxid: 82 g Cefodizim in Form farbloser Kristalle.

**Patentansprüche** für die Vertragsstaaten BE, CH, DE, FR, GB, IT, LI, LU, NL, SE

1. Kristalline, wasserlösliche Salze der 7-β-[2-(2-Aminothiazol-4-yl)-2-syn-methoximino-acetamido]-3-(5-carboxymethyl-4-methyl-1,3-thiazol-2-yl-thiomethyl)-ceph-3-em-4 carbonsäure, sowie deren Addukte und Solvate mit Wasser und/oder organischen Lösungsmitteln.

2. Verfahren zur Herstellung von kristallinen, wasserlöslichen Salzen der 7-β-[2-(2-Aminothiazol-4-yl)-2-syn-methoximino-acetamido]-3-(5-carboxymethyl-4-methyl-1,3-thiazol-2-yl-4-thiomethyl)-ceph-3-em-4-carbonsäure, sowie deren Addukten und Solvaten mit Wasser und/oder organischen Lösungsmitteln, dadurch gekennzeichnet, dass man die 7-β-[2-(2-Aminothiazol-4-yl)-2-syn-methoximino-acetamido]-3-(5-carboxymethyl-4-methyl-1,3-thiazol-2-yl-thiomethyl)-ceph-3-em-4-carbonsäure mit mindestens dem zweifachen Äquivalent einer basischen Verbindung in Wasser in Lösung bringt, mit einem mit Wasser mischbaren, organischen Lösungsmittel, wobei Methanol ausgenommen ist, versetzt, bis Kristallisation eintritt und – falls erwünscht – aus den so erhaltenen Addukten und Solvaten das organische Lösungsmittel und gegebenenfalls auch das Wasser entfernt, wobei anschliessend durch Einwirkung eines organischen Lösungsmittels auch noch die Überführung in ein Solvat erfolgen kann.

3. Gegen bakterielle Infektionen wirksame pharmazeutische Präparate, gekennzeichnet durch einen Gehalt an Cephemderivaten aus Anspruch 1.

4. Verfahren zur Herstellung von gegen bakterielle Infektionen wirksamen pharmazeutischen Präparaten, dadurch gekennzeichnet, dass ein Cephemderivat aus Anspruch 1, gegebenenfalls mit pharmazeutisch üblichen Trägerstoffen oder Verdünnungsmitteln in eine pharmazeutisch geeignete Verabreichungsform gebracht wird.

**Patentansprüche** für den Vertragsstaat AT

1. Verfahren zur Herstellung von kristallinen, wasserlöslichen Salzen der 7-β-[2-(2-Aminothiazol-4-yl)-2-syn-methoximino-acetamido]-3-(5-carboxymethyl-4-methyl-1,3-thiazol-2-yl-thiomethyl)-ceph-3-em-4-carbonsäure, sowie deren Addukten und Solvaten mit Wasser und/oder organischen Lösungsmitteln, dadurch gekennzeichnet, dass man die 7-β-[2-(2-Aminothiazol-4-yl)-2-syn-methoximino-acetamido]-3-(5-carboxymethyl-4-methyl-1,3-thiazol-2-yl-thiomethyl)-ceph-3-em-4-carbonsäure mit mindestens dem zweifachen Äquivalent einer basischen Verbindung in Wasser in Lösung bringt, mit einem mit Wasser mischbaren, organischen Lösungsmittel, wobei Methanol ausgenommen ist, versetzt, bis Kristallisation eintritt und – falls erwünscht – aus den so erhaltenen Addukten und Solvaten das organische Lösungsmittel und gegebenenfalls auch das Wasser entfernt, wobei anschliessend durch Einwirkung eines organischen Lösungsmittels auch noch die Überführung in ein Solvat erfolgen kann.

2. Verfahren zur Herstellung von gegen bakterielle Infektionen wirksamen pharmazeutischen Präparaten, dadurch gekennzeichnet, dass ein Cephemderivat aus Anspruch 1 gegebenenfalls mit pharmazeutisch üblichen Trägerstoffen oder Verdünnungsmitteln in eine pharmazeutisch geeignete Verabreichungsform gebracht wird.

**Claims** for the Contracting States BE, CH, DE, FR, GB, IT, LI, LU, NL, SE

1. Crystalline water-soluble salts of 7-β-[2-(2-aminothiazol-4-yl)-2-syn-methoximinoacetamido]-3-(5-carboxymethyl-4-methyl-1,3-thiazol-2-ylthiomethyl)ceph-3-em-4-carboxylic acid, and its adducts and solvates with water and/or organic solvents.

2. A process for the preparation of crystalline water-soluble salts of 7-β-[2-(2-aminothiazol-4-yl)-2-syn-methoximinoacetamido]-3-(5-carboxymethyl-4-methyl-1,3-thiazol-2-ylthiomethyl)ceph-3-em-4-carboxylic acid, and its adducts and solvates with water and/or organic solvents, which comprises dissolving 7-β-[2-(2-aminothiazol-4-yl)-2-syn-methoximinoacetamido]-3-(5-carboxymethyl-4-methyl-1,3-thiazol-2-ylthiomethyl)ceph-3-em-4-carboxylic acid in water with at least twice the equivalent amount of a basic compound, adding a water-miscible organic solvent – with the proviso that methanol in excepted – until crystallization occurs and, if desired, removing the organic solvent and, optionally, also the water from the adducts or solvates thus obtained, it also being possible subsequently to carry out conversion into a solvate by the action of an organic solvent.

3. Pharmaceutical formulations active against bacterial infections having a content of a cephem derivative as claimed in claim 1.

4. A process for the preparation of pharmaceutical formulations active against bacterial infections, which comprises converting a cephem derivative as claimed in claim 1 into a pharmaceutically suitable form for administration, optionally with customary pharmaceutical vehicles or diluents.

**Claims** for the Contracting State AT

1. A process for the preparation of crystalline water-soluble salts of 7-β-[2-(2-aminothiazol-4-yl)-2-syn-methoximinoacetamido]-3-(5-carboxymethyl-4-methyl-1,3-thiazol-2-ylthiomethyl)ceph-3-em-4-carboxylic acid, and its adducts and solvates with water and/or organic solvents, which comprises dissolving 7-β-[2-(2-aminothiazol-4-yl)-2-syn-methoximinoacetamido]-3-(5-carboxymethyl-4-methyl-1,3-thiazol-2-ylthiomethyl)ceph-3-em-4-carboxylic acid in water with at least twice the equivalent amount of a basic compound, adding a water-miscible organic solvent – with the proviso that methanol in excepted – until crystallization occurs and, if desired, removing the organic solvent and, optionally, also the water from the adducts or solvates thus obtained, it also being possible subsequently to carry out conversion into a solvate by the action of an organic solvent.

2. A process for the preparation of pharmaceutical formulations active against bacterial infections, which comprises converting a crystalline water-soluble salt of 7-β-[2-(2-aminothiazol-4-yl)-2-syn-methoximinoacetamido]-3-(5-carboxymethyl-4-methyl-1,3-thiazol-2-ylthiomethyl)ceph-3-em-4-carboxylic acid, or its adducts or solvates with water and/or organic solvents into a pharmaceutically suitable form for administration, optionally with customary pharmaceutical vehicles or diluents.

**Revendications** pour les Etats Contractants BE, CH, DE, FR, GB, IT, LI, LU, NL, SE

1. Sels cristallins, solubles dans l'eau, de l'acide 7-β-[2-(2-aminothiazol-4-yl)-2-syn-méthoximino-acétamido]-3-(5-carboxyméthyl-4-méthyl-1,3-thiazol-2-yl-thiométhyl)-céph-3-ème-4-carboxylique, ainsi que leurs produits d'addition et solvats avec l'eau et/ou des solvants organiques.

2. Procédé pour la préparation de sels cristallins, solubles dans l'eau, de l'acide 7-β-[2-(2-aminothiazol-4-yl)-2-syn-méthoximino-acétamido]-3-(5-carboxyméthyl-4-méthyl-1,3-thiazol-2-yl-thiométhyl)-céph-3-ème-4-carboxylique ainsi que de leurs produits d'addition et solvats avec l'eau et/ou des solvants organiques, caractérisé en ce que l'on met en solution dans de l'eau l'acide 7-β-[2-(2-aminothiazol-4-yl)-2-syn-méthoximino-acétamido]-3-(5-carboxyméthyl-4-méthyl-1,3-thiazol-2-yl-thiométhyl)-céph-3-ème-4-carboxylique avec au moins deux équivalents d'un composé basique, on ajoute un solvant organique miscible à l'eau, le méthanol étant exclu, jusqu'à ce que la cristallisation se produise et – si on le désire – on élimine le solvant organique et, éventuellement, aussi l'eau, des produits d'addition et solvats ainsi obtenus, la transformation en un solvat pouvant encore être effectuée ensuite sous l'action d'un solvant organique.

3. Compositions pharmaceutiques actives contre des infections bactériennes, caractérisée par une teneur en dérivés de céphème selon la revendication 1.

4. Procédé pour la préparation de compositions pharmaceutiques actives contre des infections bactériennes, caractérisées en ce que l'on met sous une forme d'administration pharmaceutiquement appropriée un dérivé de céphème selon la revendication 1, éventuellement avec des véhicules ou diluants pharmaceutiques usuels.

**Revendications** pour l'Etat Contractant AT

1. Procédé pour la préparation de sels cristallins, solubles dans l'eau, de l'acide 7-β-[2-(2-aminothiazol-4-yl)-2-syn-méthoximino-acétamido]-3-(5-carboxyméthyl-4-méthyl-1,3-thiazol-2-yl-thiométhyl)-céph-3-ème-4-carboxylique, ainsi que de leurs produits d'addition et solvats avec l'eau et/ou des solvants organiques, caractérisé en ce que l'on met en solution dans de l'eau l'acide 7-β-[2-(2-aminothiazol-4-yl)-2-syn-méthoximino-acétamido]-3-(5-carboxyméthyl-4-méthyl-1,3-thiazol-2-yl-thiométhyl)-céph-3-ème-4-carboxylique avec au moins deux équivalents d'un composé basique, on ajoute un solvant organique miscible à l'eau, le méthanol étant exclu, jusqu'à ce que la cristallisation se produise et – si on le désire – on élimine le solvant organique et, éventuellement, aussi l'eau, des produits d'addition et solvats ainsi obtenus, la transformation en un solvat pouvant encore être effectuée ensuite sous l'action d'un solvant organique.

2. Procédé pour la préparation de compositions pharmaceutiques actives contre des infections bactériennes, caractérisées en ce que l'on met sous une forme d'administration pharmaceutiquement appropriée un dérivé de céphème selon la revendication 1, éventuellement avec des véhicules ou diluants pharmaceutiques usuels.